(19) Europäisches Patentamt
European Patent Office
Office européen des brevets

(11) Veröffentlichungsnummer: **0 366 060 B1**

(12) **EUROPÄISCHE PATENTSCHRIFT**

(45) Veröffentlichungstag der Patentschrift: **02.03.94**

(21) Anmeldenummer: **89119704.8**

(22) Anmeldetag: **24.10.89**

(51) Int. Cl.5: **C07D 309/14**, C12P 17/06, C12P 1/06, A61K 31/35, C12N 9/99, A01N 43/16, //(C12P1/06,C12R1:47)

# (54) Glykosidase-Inhibitor Salbostatin, Verfahren zu seiner Herstellung und seine Verwendung.

(30) Priorität: **28.10.88 DE 3836675**

(43) Veröffentlichungstag der Anmeldung: **02.05.90 Patentblatt 90/18**

(45) Bekanntmachung des Hinweises auf die Patenterteilung: **02.03.94 Patentblatt 94/09**

(84) Benannte Vertragsstaaten: **AT BE CH DE ES FR GB GR IT LI LU NL SE**

(56) Entgegenhaltungen:
**US-A- 4 062 950**
**US-A- 4 065 557**
**US-A- 4 254 256**

(73) Patentinhaber: **HOECHST AKTIENGESELLSCHAFT**
**D-65926 Frankfurt(DE)**

(72) Erfinder: **Vértesy, Lászlo, Dr.**
**Eppenhainer Weg 6**
**D-6239 Eppstein/Taunus(DE)**
Erfinder: **Fehlhaber, Hans-Wolfram, Dr.**
**Thomas-Mann-Strasse 5a**
**D-6270 Idstein/Taunus(DE)**
Erfinder: **Schulz, Arno, Dr.**
**Stauffenstrasse 22**
**D-6234 Hatterheim am Main(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft das biologisch aktive Pseudodisaccharid Salbostatin, seine physiologisch verträglichen Salze, Verfahren zu seiner Herstellung sowie seine Verwendung in der Pharmazie und im Pflanzenschutz.

Salbostatin ist ein basisches, nicht reduzierendes Pseudodisaccharid, welches durch die Formel I und durch die chemischen bzw. physikalischen Eigenschaften gekennzeichnet ist.

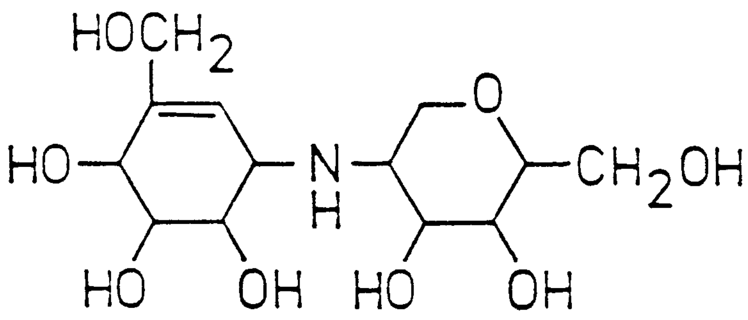

I

Basische Pseudodisaccharide bzw. Pseudooligosaccharide mit enzymhemmender Wirkung sind in der Literatur verschiedentlich beschrieben worden (s. US-Patente 4 062 950, 4 065 557, 4 254 256, darüber hinaus E. Truscheit et al. Angew. Chem. 93, 738-755, (1981)). Diese haben saccharase- und maltasehemmende oder antimikrobielle Eigenschaften und können z.B. bei der Behandlung des Diabetes mellitus bzw. als Antibiotika eingesetzt werden.

Die erfindungsgemäße Substanz Salbostatin weist eine Wirksamkeit als Glykosidase-Inhibitor auf und eignet sich deshalb zum Einsatz in der Pharmazie und im Pflanzenschutz.

Das Salbostatin besitzt folgende Konfiguration:

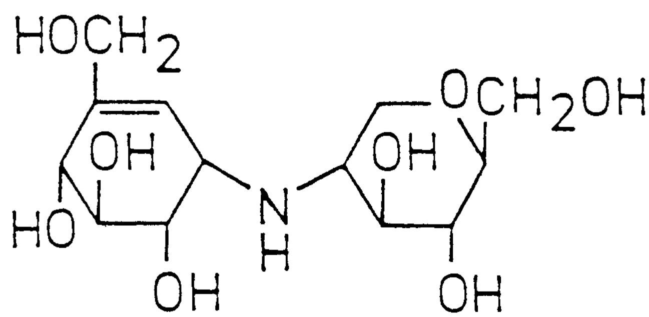

Ein derartiger basischer, cyclischer, nicht reduzierender Ether ist bisher nicht beschrieben worden.

Weiterhin gehören zum Erfindungsgegenstand physiologisch verträgliche Salze des Salbostatins, insbesondere Säureadditionssalze, wie z.B. das Hydrochlorid, die sich auf allgemein bekannte Weise herstellen lassen.

Die Erfindung betrifft ferner ein Verfahren zur Herstellung des Salbostatins, das dadurch gekennzeichnet ist, daß

a) der im Handel erhältliche Mikroorganismus Streptomyces albus (ATCC 21838), sowie seine Varianten und Mutanten in einem Nährmedium kultiviert werden bis sich die Verbindung der Formel I in der Kultur anhäuft, und die Verbindung gegebenenfalls

b) isoliert, gereinigt und derivatisiert wird.

In einer Nährlösung, die eine Kohlenstoffquelle und eine Stickstoffquelle sowie die für diese Zwecke üblichen anorganischen Salze enthält, produziert Streptomyces albus, ATCC 21838, unter anderem das Salbostatin.

Anstelle des Stammes ATCC 21838 können natürlich auch seine Mutanten und/oder Varianten eingesetzt werden, soweit sie die erfindungsgemäße Verbindung synthetisieren. Solche Mutanten können in an sich bekannter Weise durch Ultraviolett- oder Röntgenstrahlen, oder chemische Mutagene, wie beispielsweise Ethansulfonsäuremethylester (Ethylmethylsulfonat, EMS) oder 2-Hydroxy-4-methoxybenzophenon (MOB), erzeugt werden.

Als bevorzugte Kohlenstoffquellen für die aerobe Fermentation eignen sich z.B. assimilierbare Kohlenhydrate und Zuckeralkohole, wie Glucose, Lactose oder D-Mannit sowie kohlenhydrathaltige Naturprodukte, wie Malzextrakt sowie Öle und Fette. Als stickstoffhaltige Nährstoffe kommen u.a. in Betracht: Aminosäuren, Peptide und Proteine sowie deren Abbauprodukte wie Peptone oder Tryptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. An anderen anorganischen Salzen kann die Nährlösung beispielsweise Chloride, Carbonate, Sulfate oder Phosphate der Alkali- oder Erdalkalimetalle oder Spurenelemente wie z.B. Eisen, Zink, Kobalt und Mangan enthalten.

Die Bildung des Salbostatins verläuft besonders gut in einer Nährlösung, die 0,1 bis 15 % Nährstoffbestandteile, wie Sojamehl, Sojaöl und Mannit, insbesondere jeweils 0,1 bis 3 %, bezogen auf das Gewicht der gesamten Nährlösung enthält. Die Fermentation erfolgt vorzugsweise aerob, beispielsweise submers unter Schütteln oder Rühren in Schüttelkolben oder Fermentern, gegebenenfalls unter Einführen von Luft oder Sauerstoff. Die Fermentation kann in einem Temperaturbereich von etwa 18 bis 35 °C, vorzugsweise bei etwa 25 bis 30 °C, insbesondere bei 28 bis 30 °C erfolgen. Der pH-Bereich sollte zwischen 6 und 8 liegen, vorteilhaft zwischen 6,5 und 7,5. Unter diesen Bedingungen zeigt die Kulturbrühe im allgemeinen nach 6 bis 15 Tagen eine nennenswerte Konzentration an Salbostatin.

Es ist vorteilhaft, in mehreren Stufen zu kultivieren, d.h. man stellt zunächst eine oder mehrere Vorkulturen in einem flüssigen Nährmedium her, die dann in das eigentliche Produktionsmedium, die Hauptkultur, beispielsweise im Volumenverhältnis 1:10, überimpft werden. Die Vorkultur erhält man z.B., indem man ein versportes Mycel in eine Nährlösung überimpft und etwa 80 bis 400 Stunden wachsen läßt. Das versporte Mycel kann erhalten werden, indem man den Stamm etwa 12 Tage auf einem festen oder flüssigen Nährboden, beispielsweise Hefe-Malz-Agar, wachsen läßt.

Der Fermentationsverlauf kann anhand des pH-Wertes der Kultur oder dem Mycelvolumen oder durch Ausprüfen der biologischen AktIVItät überwacht werden. Das Salbostatin ist sowohl im Mycel als auch im Kulturfiltrat enthalten. Die Hauptmenge des Salbostatins ist jedoch im allgemeinen im Kulturfiltrat zu finden.

Die Isolierung der genannten Verbindungen aus dem Kulturmedium erfolgt nach bekannten Methoden unter Berücksichtigung der chemischen, physikalischen und biologischen Eigenschaften der Produkte, wie beispielsweise Chromatographie an Ionenaustauschern, Molekularsieben, Adsorptionsharzen und Reversed-Phase-Trägern, durch Lösungsmittelfällung, Reverseosmose und andere.

Vorteilhaft ist es weiterhin, die wäßrige Phase vom Mycel, beispielsweise durch Filtration oder Zentrifugation, zu trennen und das Salbostatin aus den jeweiligen Phasen zu isolieren und zu reinigen.

Ein bevorzugtes Verfahren besteht darin, daß das Kulturfiltrat zur Entfettung mit Butanol extrahiert und die wäßrige Phase im Vakuum eingeengt wird. Das mit der 2- bis 5-fachen Menge Methanol verdünnte wäßrige Konzentrat wird durch Zentrifugation von den ausgefallenen hochmolekularen Stoffen befreit. Aus dem wäßrig-methanolischen Überstand werden dann mit stark sauren Ionenaustauschern wie z.B. (R)Dowex 50 WX 2 zunächst die basischen Bestandteile isoliert. Anschließend werden mit einem Anionenaustauscher wie z.B. (R)Amberlite IRA-68 hiervon die amphoteren Substanzen entfernt. Die verbleibende basische Fraktion enthält das Salbostatin. Nach Entsalzung mit Hilfe eines Molekularsiebes wie z.B. (R)Sephadex LH-2 wird durch Auftrennung mittels Ionenaustauscherchromatographie und erneuter Entsalzung das reine Salbostatin gewonnen. $^1$H-NMR-Spektren des reinen Salbostatins zeigt die Figur 1. Spektrum a) wurde in $d_6$-DMSO bei 400 MHz und 300 K aufgenommen; die chemische Verschiebung ist auf TMS bezogen. Spektrum b) wurde in $d_6$-DMSO unter Zusatz von 1 % $H_2O$ bei 300 K aufgenommen.

Das erfindungsgemäße Salbostatin eignet sich als Glykosidase-Inhibitor zur Therapie von Stoffwechselerkrankungen des Menschen und von warmblütigen Tieren.

Als Inhibitor der Trehalase eignet sich das Salbostatin als Pflanzenschutzmittel. Die Trehalose ist ein wichtiger Energiespeicherstoff für Insekten, Pilze und auch in der Pflanzenwelt, z.B. in einigen Pollenkörpern. In die Stoffwechselvorgänge der Trehalose die für Warmblüter kaum Bedeutung hat, ist stets das hydrolytische Enzym Trehalase eingeschaltet. Hemmt man dieses Enzym, so kann man selektiv die Organismen treffen, die trehalaseabhängig sind.

Die Inhibition des Enzyms Trehalase durch das Salbostatin ist sehr effektiv. Bereits in $10^{-7}$ bis $10^{-8}$ molarer Salbostatin-Lösung wird das Enzym zu 50 % gehemmt. Nähere enzymkinetische Untersuchungen zeigten, daß Salbostatin die Trehalase kompetitiv in Bezug auf Trehalose hemmt. Die Inhibitorkonstante Ki beträgt 1,8 x $10^{-7}$ M.

Die vorliegende Erfindung betrifft weiterhin pharmazeutische Präparate sowie Pflanzenschutzmittel, die einen Gehalt an einer erfindungsgemäßen Verbindung aufweisen und die nach allgemein bekannten Methoden hergestellt werden können (vgl. z.B. EP 0 240 853 und EP 0 224 024).

**Trehalasetest:**

Der Trehalasetest basiert auf der kolorimetrischen Bestimmung von D-Glucose, die durch die Trehalase aus Trehalose freigesetzt wird.

Trehalase (Sigma Nr. T 8778) wurde vor Testbeginn 1:1000 in 20mM Phosphatpuffer pH 6,5 verdünnt. Trehalose wurde in 0,1M Konzentration in 0,1M 2-(N-Morpholino)ethansulfonsäure gelöst.

Zu Testbeginn wurden je 100 µl $H_2O$, Trehalose und Trehalase zusammengegeben und 30 Min. bei 37 °C inkubiert. Die Reaktion wurde durch Erhitzen des Ansatzes auf 100 °C gestoppt. Der Kontrollansatz wurde sofort (0 Min.) durch Kochen abgestoppt. Salbostatin wurde gegebenenfalls in verschiedenen Konzentrationen anstelle des Wassers den Reaktionsansätzen zugefügt.

Die aus der Trehalose freigesetzte Glucose wurde kolorimetrisch bestimmt. Dazu wurde die Glucose durch Glucose-Oxidase umgesetzt und das entstehende $H_2O_2$ zur Oxidation von o-Dianisidin verwendet. Das oxidierte o-Dianisidin wurde im Photometer bestimmt. Alle oben erwähnten Reagentien zur Glucosebestimmung wurden dem Glucose-Kit der Firma Sigma (Sigma Chemie GmbH, Deisenhofen, Deutschland) entnommen (Nr. 510-DA) und der Test entsprechend dem mitgelieferten Protokoll durchgeführt.

**Beispiel 1**

**Fermentative Gewinnung des Salbostatins (2000 l)**

Zur fermentativen Gewinnung des erfindungsgemäßen Glukosidase-Inhibitors erfolgte die Anzucht des produzierenden Mikroorganismus Streptomyces albus ATCC 21838 - wie in der Mikrobiologie üblich - aus einer gefriergetrockneten Dauerform dieses Stammes. Die Anzucht erfolgt zunächst auf einem festen Nährboden in sterilen Petri-Schalen. Vereinzelte Kolonien wurden anschließend in Schrägröhrchen weiter kultiviert und mit diesen Kulturen die für die Fermentation notwendige Massenproduktion von Sporen in Roux-Flaschen durchgeführt.

Agarmedium für Passagen auf festen Nährböden:

| | |
|---|---|
| Dextrin | 15,0 g/l |
| Rohrzucker | 2,0 g/l |
| Fleischextrakt | 1,0 g/l |
| Hefeextrakt | 2,0 g/l |
| Kochsalz | 0,5 g/l |
| $K_2HPO_4$ | 0,5 g/l |
| $FeSO_4 \cdot 7H_2O$ | 0,01 g/l |
| Agar-Agar | 2,0 g/l |
| pH-Wert | 7,3 |
| Sterilisation bei | 120 °C, 20 Minuten |
| Inkubation bei | 30 °C, 9 Tage |

Die Sporenabschwemmung einer Rouxflasche wurde in 100 ml sterilem Wasser aufgenommen und diente als Inokulum für die Herstellung der ersten submersen vegetativen Fermenations-Vorstufe (Arbeitsvolumen 1,2 l).

Vorstufen-Medium:

| | |
|---|---|
| lösliche Stärke | 4,0 g/l |
| Glucose | 1,0 g/l |
| Casein-Pepton | 1,0 g/l |
| Cornsteep flüssig | 0,4 g/l |
| Sojamehl | 0,4 g/l |
| $(NH_4)_2SO_4$ | 0,8 g/l |
| pH-Wert | 8,3 |
| Sterilisation bei | 120 °C, 20 Minuten |
| Inkubation bei | 18 °C, 2 Tage an einer Schüttelmaschine mit 150 UpM und 5 cm Amplitude |

Nach 48 Stunden wurde der inhalt einer Fernbachflasche mit der so erhaltenen ersten Vorkultur (s.o.) als Inokulum für die 200 l betragende zweite Vorstufe eingesetzt, wobei auch hier das Vorstufen-medium (s.o.) verwendet wurde. Die Sterilisationszeit betrug 30 Minuten. Die Inkubation erfolgte 48 Stunden lang bei 28 °C unter Rühren mit einer Umfangsgeschwindigkeit von 5 m/sec und einer Belüftungsrate von 0,1 vvm.

Der Inhalt der zweiten Vorkultur diente als Inokulum für die Hauptfermentation.

Hauptfermentationsmedium:

| | |
|---|---|
| Ernußmehl | 30,0 g/l |
| Cornsteep, fest | 10,0 g/l |
| Maisstärke | 20,0 g/l |
| Dextrin | 40,0 g/l |
| $(NH_4)_2SO_4$ | 5,0 g/l |
| $MgSO_4 \cdot 7H_2O$ | 5,0 g/l |
| $CaCO_3$ | 8,0 g/l |
| pH-Wert | 6,8 |
| Sterilisation bei | 120 °C, 50 Minuten |

Die Hauptfermentation wurde bei 28 °C unter Rühren mit einer Umfangsgeschwindigkeit von 4 m/sec. und einer Belüftungsrate von 0,6 vvm durchgeführt. Die Bildung der erfindungsgemäßen Inhibitoren begann nach 4 Tagen und erreichte ihr Maximum nach 10 bis 12 Tagen. Nach Erreichen des Fermentations-Maximums wurde der Fermenter abgeerntet. Die Ausbeute an Salbostatin der Formel I betrug zwischen 0,5 und 5 mg/l Kulturlösung.

**Beispiel 2**

**Isolierung des Salbostatins aus der Kulturlösung**

2000 l Fermentationslösung gemäß Beispiel 1 wurden mit Hilfe einer Filterpresse von der Zellmasse befreit und die klare Flüssigkeit zweimal mit je 600 l n-Butanol extrahiert. Anschließend konzentrierte man die entfettete wäßrige Phase im Vakuum in einem Fallstromverdampfer auf 150 l und verdünnte dann dieses Konzentrat mit 600 l Methanol. Hierbei entstand ein heller, flockiger Niederschlag, der durch Zentrifugieren abgeschieden und verworfen wurde. Der wäßrigmethanolische Überstand wurde erneut im Vakuum eingeengt und zu 18 kg einer braunen, klebrigen Masse getrocknet. Anschließend adsorbierte man im wäßrigen Medium auf 70 l Kationenaustauscher (R)Dowex 50 WX 2 (Säureform), wusch den beladenen Austauscher mit reinem Wasser und desorbierte die Inhibitoren mit 0,5 M Ammoniumhydroxidlösung. Die trehalasehemmenden Eluate wurden gesammelt und gefriergetrocknet (3,5 kg). Hieraus wurden durch analoge Arbeitsweise an 70 l Anionenaustauscher (R)Amberlite IRA-68 die amorphen Verbindungen abgetrennt. Das Salbostatin war im Durchlauf der Säule enthalten. Die Gefriertrocknung des inhibitorisch aktiven Durchflusses erbrachte 850 g Trockenprodukt. Die Gelchromatographie an Sephadex LH-20 sowie die Gefriertrocknung des aktiven Materials ergab 62 g Rohsalbostatin.

30 g dieses Materials wurden anschließend auf eine S-Sepharose Fast Flow-Säule von 2,4 l Inhalt aufgetragen. Der Kationenaustauscher war zuvor mit Ammoniumacetatpuffer, pH 3,1, equilibriert worden.

Die Elution des Salbostatins erfolgte mit einem 0 bis 0,6molaren Ammoniumacetatgradienten. Die trehalasehemmenden Fraktionen wurden gefriergetrocknet und anschließend auf Sephadex LH-20 und nochmals auf Fractogel TSK HW-40 entsalzt und gefriergetrocknet. Es resultierten 520 mg reines, amorphes weißes Salbostatin. Die optische Drehung beträgt $[\alpha]_D^{20}$ = + 115° (c = 1 in Wasser).

**Patentansprüche**

**Patentansprüche für folgende Vertragsstaaten : AT, BE, CH, DE, FR, GB, IT, LI, LU, NL, SE**

1. Eine Verbindung der Formel I

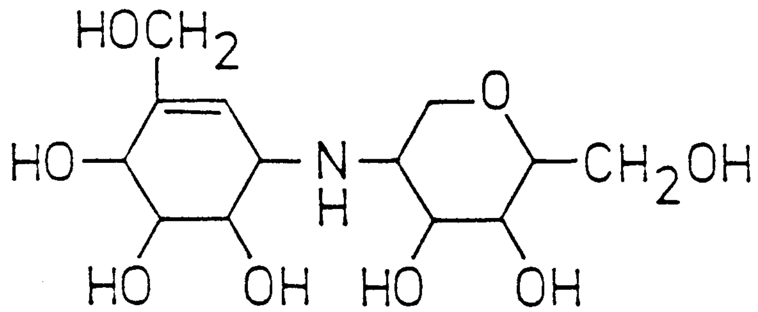

sowie ihre physiologisch verträglichen Salze.

2. Verfahren zur Herstellung einer Verbindung der Formel I gemäß Anspruch 1, dadurch gekennzeichnet, daß man den Mikroorganismus Streptomyces albus ATCC 21838 oder einen seiner Mutanten oder Varianten kultiviert und die Verbindung der Formel I aus dem Kulturfiltrat und/oder dem Mycel isoliert und reinigt.

3. Verfahren gemäß Anspruch 2, dadurch gekennzeichnet, daß die Kultivierung unter submersen, aeroben Bedingungen stattfindet.

4. Pharmazeutisches Präparat, gekennzeichnet durch einen Gehalt an einer Verbindung gemäß Anspruch 1.

5. Verwendung einer Verbindung gemäß Anspruch 1 als Glykosidase-Hemmer.

6. Eine Verbindung gemäß Anspruch 1 zur Anwendung als Arzneimittel.

7. Pflanzenschutzmittel, gekennzeichnet durch einen Gehalt an einer Verbindung gemäß Anspruch 1.

8. Verwendung einer Verbindung gemäß Anspruch 1 als Wirkstoff in einem Pflanzenschutzmittel.

9. Verfahren zum Schutz von Pflanzen vor Insekten und/oder schädlichen Pilzen, dadurch gekennzeichnet, daß man die Pflanzen mit einer Verbindung gemäß Anspruch 1 behandelt.

10. Verwendung einer Verbindung gemäß Anspruch 1 zur Herstellung von Arzneimitteln zur Therapie von Stoffwechselerkrankungen.

**Patentansprüche für folgende Vertragsstaaten : ES, GR**

1. Verfahren zur Herstellung einer Verbindung der Formel I

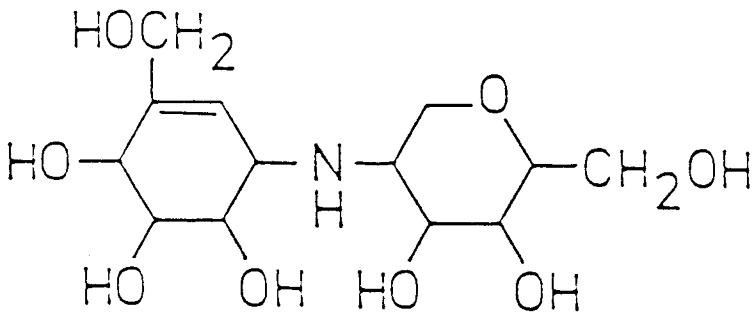

I

dadurch gekennzeichnet, daß man den Mikroorganismus Streptomyces albus ATCC 21838 oder einen seiner Mutanten oder Varianten kultiviert und die Verbindung der Formel I aus dem Kulturfiltrat und/oder dem Mycel isoliert und reinigt.

2. Verfahren gemäß Anspruch 1, dadurch gekennzeichnet, daß die Kultivierung unter submersen, aeroben Bedingungen stattfindet.

3. Verfahren zur Herstellung eines pharmazeutischen Präparates, dadurch gekennzeichnet, daß man eine Verbindung herstellt gemäß Anspruch 1 und gegebenenfalls mit üblichen Hilfs- und/oder Trägerstoffen in eine geeignete Darreichungsform bringt.

4. Verwendung einer Verbindung gemäß Anspruch 1 als Glykosidase-Hemmer.

5. Verwendung einer Verbindung gemäß Anspruch 1 zur Herstellung von Arzneimitteln zur Therapie von Stoffwechselerkrankungen.

6. Pflanzenschutzmittel, gekennzeichnet durch einen Gehalt an einer Verbindung gemäß Anspruch 1.

7. Verfahren zur Herstellung eines Pflanzenschutzmittels, dadurch gekennzeichnet, daß man eine Verbindung gemäß Anspruch 1 herstellt und mit üblichen Hilfs- und Zusatzstoffen in ein Pflanzenschutzmittel überführt.

8. Verwendung einer Verbindung gemäß Anspruch 1 als Wirkstoff in einem Pflanzenschutzmittel.

9. Verfahren zum Schutz von Pflanzen vor Insekten und/oder schädlichen Pilzen, dadurch gekennzeichnet, daß man die Pflanzen mit einer Verbindung gemäß Anspruch 1 behandelt.

## Claims

**Claims for the following Contracting States : AT, BE, CH, DE, FR, GB, IT, LI, LU, NL, SE**

1. A compound of the formula I

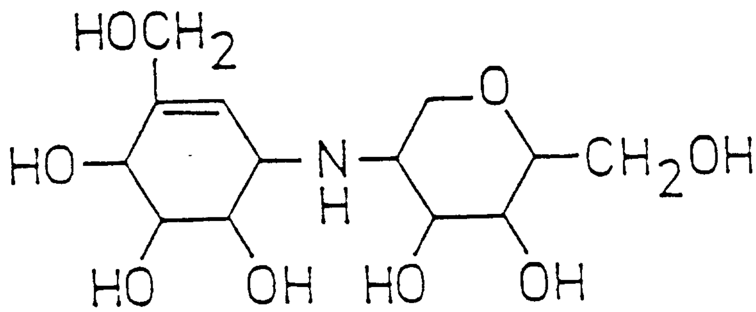

and its physiologically acceptable salts.

2. A process for the preparation of a compound of the formula I as claimed in claim 1, which comprises cultivating the microorganism Streptomyces albus ATCC 21838 or one of its mutants or variants, isolating the compound of the formula I from the culture filtrate and/or the mycelium, and purifying it.

3. The process as claimed in claim 2, wherein the cultivation is carried out under submerse, aerobic conditions.

4. A pharmaceutical preparation, which contains a compound as claimed in claim 1.

5. The use of a compound as claimed in claim 1 as a glycosidase inhibitor.

6. A compound as claimed in claim 1 for use as a pharmaceutical.

7. A plant protection agent, containing a compound as claimed in claim 1.

8. The use of a compound as claimed in claim 1 as the active ingredient in a plant protection agent.

9. A method of protecting plants from insects and/or harmful fungi, which comprises treating the plants with a compound as claimed in claim 1.

10. The use of a compound as claimed in claim 1 for the production of pharmaceuticals for the therapy of metabolic diseases.

**Claims for the following Contracting States : ES, GR**

1. A process for the preparation of a compound of the formula I

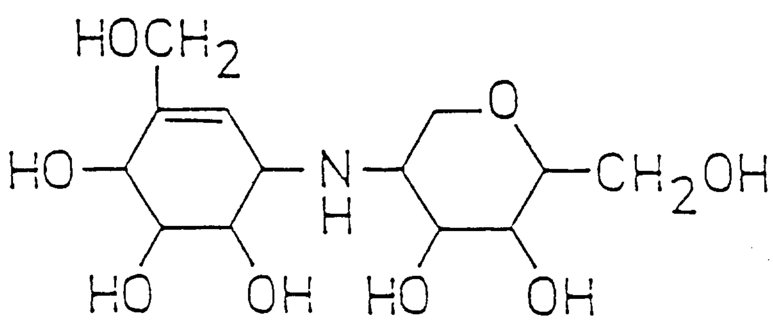

I

which comprises cultivating the microorganism Streptomyces albus ATCC 21838 or one of its mutants or variants, isolating the compound of the formula I from the culture filtrate and/or the mycelium, and purifying it.

**2.** The process as claimed in claim 1, wherein the cultivation is carried out under submerse, aerobic conditions.

**3.** A method of producing a pharmaceutical preparation, which comprises preparing a compound as claimed in claim 1 and bringing it into a suitable administration form, if desired together with conventional auxiliaries and/or excipients.

**4.** The use of a compound as in claim 1 as a glycosidase inhibitor.

**5.** The use of a compound as in claim 1 for the production of pharmaceuticals for the therapy of metabolic diseases.

**6.** A plant protection agent, containing a compound as in claim 1.

**7.** A method of producing a plant protection agent, which comprises preparing a compound as claimed in claim 1 and converting it into a plant protection agent together with conventional auxiliaries and additives.

**8.** The use of a compound as in claim 1 as the active ingredient in a plant protection agent.

**9.** A method of protecting plants from insects and/or harmful fungi, which comprises treating the plants with a compound as in claim 1.

**Revendications**

**Revendications pour les Etats contractants suivants : AT, BE, CH, DE, FR, GB, IT, LI, LU, NL, SE**

**1.** Composé de formule I:

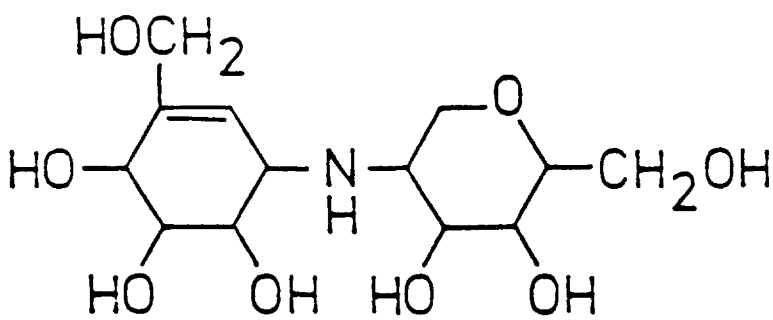

ainsi que ses sels physiologiquement compatibles.

**2.** Procédé de préparation d'un composé de formule I selon la revendication 1, caractérisé en ce qu'on cultive le microorganisme Streptomyces albus ATCC 21838 ou un des ses mutants ou variantes, on isole du filtrat de culture et/ou du mycélium le composé de formule I et on le purifie.

**3.** Procédé selon la revendication 2, caractérisé en ce que la culture a lieu dans des conditions aérobies en submersion.

**4.** Préparation pharmaceutiques, caractérisée en ce qu'elle contient une certaine teneur d'un composé selon la revendication 1.

**5.** Utilisation d'un composé selon la revendication 1 comme inhibiteur de glycosidase.

6. Utilisation d'un composé selon la revendication 1 comme médicament.

7. Agent phytosanitaire , caractérisé en ce qu'il contient une certaine teneur d'un composé selon la revendication 1.

8. Utilisation d'un composé selon la revendication 1 comme principe actif dans un agent phytosanitaire.

9. Procédé pour protéger les plantes des insectes et/ou des champignons toxiques, caractérisé en ce qu'on traite les plantes avec un composé selon la revendication 1.

10. Utilisation d'un composé selon la revendication 1 pour la fabrication de médicaments pour traiter les troubles du métabolisme.

**Revendications pour les Etats contractants suivants : ES, GR**

1. Procédé de préparation d'un composé de formule I:

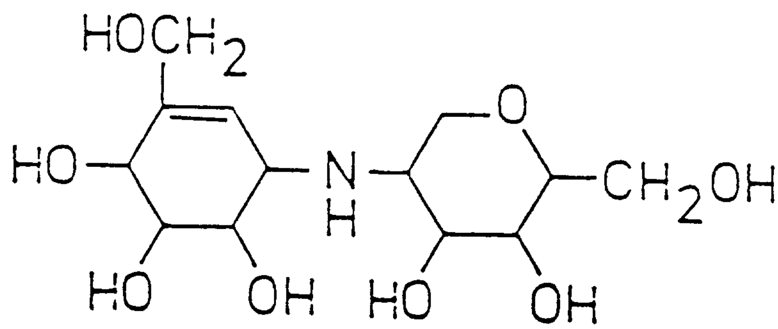

I

caractérisé en ce qu'on cultive le microorganisme Streptomyces albus ATCC 21838 ou un des ses mutants ou variantes, on isole du filtrat de culture et/ou du mycélium le composé de formule I et on le purifie.

2. Procédé selon la revendication 1, caractérisé en ce que la culture a lieu dans des conditions aérobies en submersion.

3. Procédé de fabrication d'une préparation pharmaceutique, caractérisé en ce qu'on prépare un composé selon la revendication 1 et qu'on le met sous une forme d'administration appropriée, éventuellement avec des adjuvants et/ou véhicules usuels.

4. Utilisation d'un composé selon la revendication 1 comme inhibiteur de glycosidase.

5. Utilisation d'un composé selon la revendication 1 pour la fabrication de médicaments pour traiter les troubles du métabolisme.

6. Agents phytosanitaires, caractérisé en ce qu'ils contiennent une certaine teneur d'un composé selon la revendication 1.

7. Procédé de fabrication d'un agent phytosanitaire, caractérisé en ce qu'on prépare un composé selon la revendication 1 et qu'on le transforme en un agent phytosanitaire à l'aide des adjuvants et additifs habituels.

8. Utilisation d'un composé selon la revendication 1 comme principe actif dans un agent phytosanitaire.

9. Procédé pour protéger les plantes des insectes et/ou des champignons toxiques, caractérisé en ce qu'on traite les plantes avec un composé selon la revendication 1.

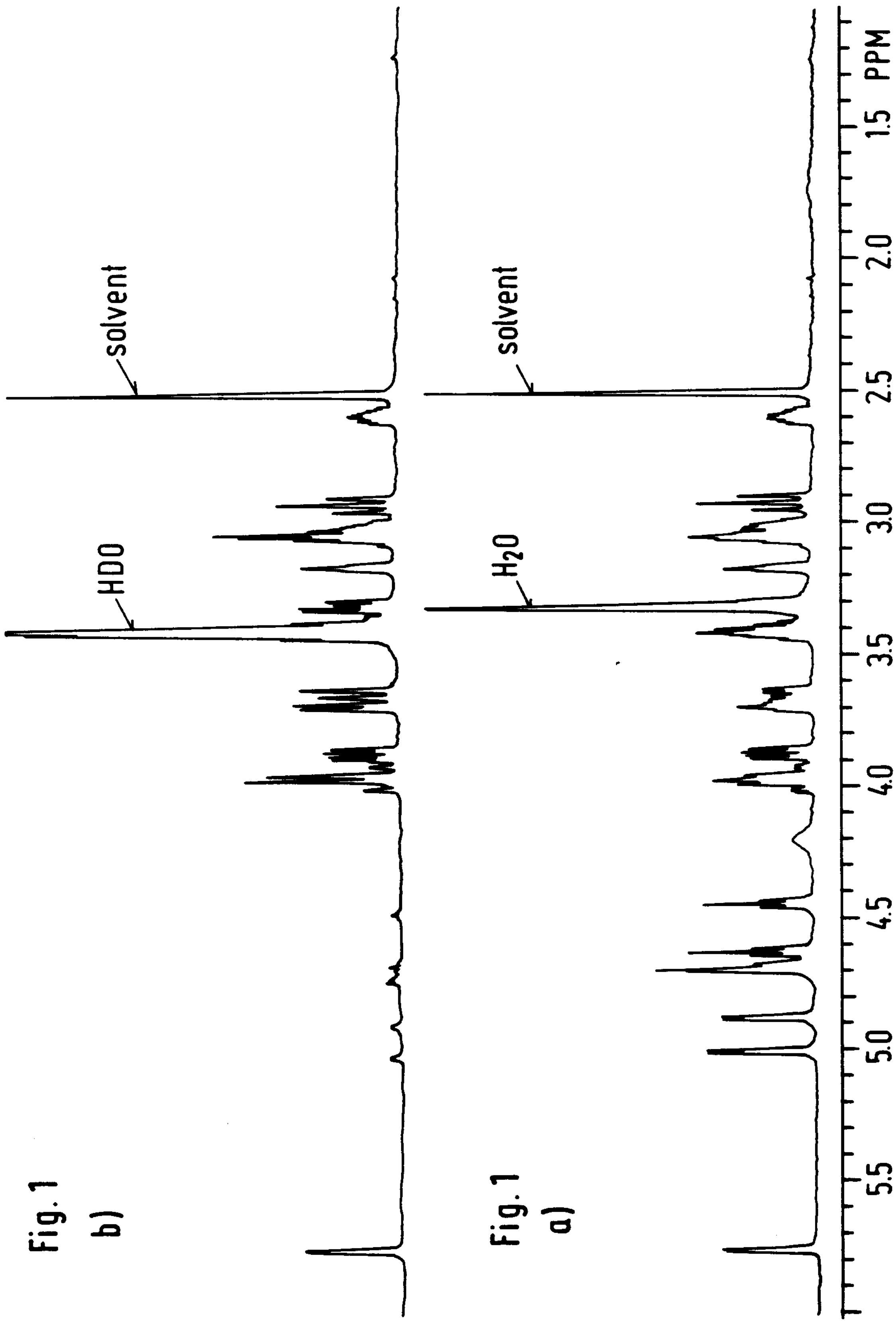


Fig. 1 b)

Fig. 1 a)